(19) Europäisches Patentamt
European Patent Office
Office européen des brevets

(11) EP 4 047 066 A1

(12) EUROPEAN PATENT APPLICATION
published in accordance with Art. 153(4) EPC

(43) Date of publication:
**24.08.2022 Bulletin 2022/34**

(21) Application number: **20875820.1**

(22) Date of filing: **16.10.2020**

(51) International Patent Classification (IPC):
***C09J 133/04*** *(2006.01)* ***C09J 7/38*** *(2018.01)*
***C09J 11/06*** *(2006.01)*

(52) Cooperative Patent Classification (CPC):
**C08F 220/1808; C09J 7/385;** C09J 2203/318;
C09J 2301/312; C09J 2301/416

(86) International application number:
**PCT/KR2020/014156**

(87) International publication number:
**WO 2021/075901 (22.04.2021 Gazette 2021/16)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **16.10.2019 KR 20190128580**
**16.10.2019 KR 20190128581**

(71) Applicant: **Koza Novel Materials Korea Co., Ltd.**
**Seocho-gu**
**Seoul**
**06627 (KR)**

(72) Inventors:
- **HONG, Jae Sung**
  **Daejeon 34122 (KR)**
- **YOON, Hu Young**
  **Daejeon 34122 (KR)**
- **MIN, Jin Seo**
  **Daejeon 34122 (KR)**
- **HWANG, Nam Ick**
  **Daejeon 34122 (KR)**
- **SONG, Ho Kyung**
  **Daejeon 34122 (KR)**
- **KIM, Woo Yeon**
  **Daejeon 34122 (KR)**

(74) Representative: **Plasseraud IP**
**66, rue de la Chaussée d'Antin**
**75440 Paris Cedex 09 (FR)**

# (54) CURABLE COMPOSITION

(57) The present application may provide a curable composition having excellent step absorptivity, and step embedding and punching properties, preventing white turbidity, and being capable of implementing a low dielectric constant, and a use thereof.

Even if such a curable composition according to the present application is applied to a stepped substrate or the like, it may prevent lifting or delayed bubbles from being generated in the step portion, and simultaneously, it may maintain the effect of suppressing white turbidity in high temperature/high humidity environments, have a low dielectric constant and improve the punching performance and handling properties. In addition, the pressure-sensitive adhesive film comprising the cured product formed from the curable composition may have excellent tensile strength.

Figure 1

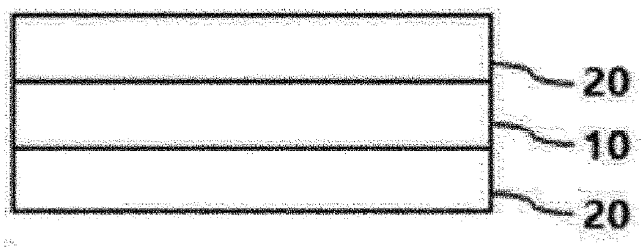




Processed by Luminess, 75001 PARIS (FR)

## Description

### Technical Feld

This application claims the benefit of priority based on Korean Patent Application Nos. 10-2019-0128580 and 10-2019-0128581 filed on October 16, 2019, the disclosures of which are incorporated herein by reference in their entirety.
The present application relates to a curable composition.

### Background Art

An OCA (optically clear adhesive) or OCR (optically clear rein) is a material applied to a display, and is often used in, for example, a display for mobile devices or vehicles.
The OCA or OCR is often applied to a stepped surface such as a bezel. Therefore, even when applied to a stepped surface, step absorptivity or step embedding properties, which can prevent the occurrence of lifting or delayed bubbles due to the step difference, is required for the OCA or OCR.
However, the step absorptivity is a physical property opposite to punching properties. The OCA or OCR is cut to the required size before application, and excellent punching properties are required for efficient cutting in this process.
In general, when a large number of polar functional groups such as a hydroxy group are included in the OCA or OCR, the punching properties increase due to an increase in cohesive force, but in this case, the step absorptivity and the step embedding properties are deteriorated.
Also, the polar functional group can play a role in preventing a white turbidity phenomenon capable of occurring in the OCA or OCR, where if the ratio of polar functional groups is reduced in consideration of step absorptivity and step embedding properties, white turbidity or the like occurs, so that the optical properties of the OCR or OCA may be lowered.
In addition, a low dielectric constant may be required for the OCA or OCR depending on an application requiring touch sensitivity, where the dielectric constant generally increases in approximately proportion to the amount of the polar functional group.
Therefore, it is not an easy task to form an OCA or OCR with a low dielectric constant having excellent step absorptivity, step embedding properties and punching properties at the same time and preventing white turbidity.

## Disclosure

### Technical Problem

The present application provides a curable composition that can be used as a so-called OCA or OCR. It is an object of the present application to provide a curable composition which has excellent step absorptivity, step embedding properties and punching properties, prevents white turbidity, and is capable of implementing a low dielectric constant, and a use thereof.

### Technical Solution

In the case where the measured temperature affects the result among physical properties mentioned in this specification, the relevant physical properties are physical properties measured at room temperature, unless otherwise specified. The term room temperature is a natural temperature without warming or cooling, which is usually a temperature within the range of about 10°C to 30°C, or a temperature of about 23°C or about 25°C or so. In addition, unless specifically stated otherwise in this specification, the unit of temperature is °C.
In the case where the measured pressure affects the result among physical properties mentioned in this specification, the relevant physical properties are physical properties measured at normal pressure, unless otherwise specified. The term normal pressure is a natural pressure without being pressurized or depressurized, where usually about 1 atmosphere or so is referred to as normal pressure.
The present application relates to a curable composition. In one example, the curable composition may be a pressure-sensitive adhesive composition. The term pressure-sensitive adhesive composition means a composition capable of forming a pressure-sensitive adhesive before or after curing. In one example, the pressure-sensitive adhesive composition of the present application may be used as an OCA (optically clear adhesive) or OCR (optically clear resin).
The curable composition or the pressure-sensitive adhesive composition may be, for example, a semi-curing type composition. The semi-curing type curable composition may mean a composition that can be further cured (may be referred to as secondary curing or post-curing) after primary curing. In one example, such a semi-curing type composition may be applied to a stepped surface in a state subjected to primary curing, and then fully cured through post-curing.

The curable composition of the present application may be a solventless curable composition. The term solventless curable composition is a composition which does not contain a solvent (aqueous solvent and organic solvent) substantially. Therefore, the content of the aqueous and organic solvents in the curable composition may be 1 weight% or less, 0.5 weight% or less, 0.1 weight% or less, or substantially 0 weight%.

The curable composition may comprise at least a polymer component and a curing agent.

The polymer component included in the curable composition may be a syrup component. The syrup component may comprise an oligomer or polymer component formed by polymerization of two or more monomers, and a monomer component. In one example, such a syrup component may be formed by so-called partial polymerization, or may be prepared by adding a monomer to a fully polymerized or partially polymerized polymer. That is, when a monomer composition according to a desired composition is partially polymerized, some monomers are polymerized to form the oligomer or polymer and the remaining monomers remain, whereby the syrup component may be formed. In another example, a syrup-type polymer component may also be formed by adding a monomer component to the partially or fully polymerized polymer. Therefore, in this specification, the term monomer unit described below may mean a monomer that exists in a state in which the oligomer or polymer is formed in the polymer component, or a monomer that is not polymerized and is included in the syrup component.

The curable composition may exhibit a punching factor (F) in a range of about 25 to 100 gf/μm before or after curing.

In the present application, the punching factor (F) has a unit of gf/μm, and is a physical quantity determined by the following equation 1.

[Equation 1]

$$F = 10 \times (A/S)$$

In Equation 1, F is a punching factor of the curable composition or its cured product (in the case of the semi-curing type, primary cured product or post-cured product); A is a toughness value (unit: gf·mm) of the curable composition or its cured product (in the case of the semi-curing type, primary cured product or post-cured product); and S is an area of the surface of the curable composition or its cured product (in the case of the semi-curing type, primary cured product or post-cured product) which is perpendicular to the tensile direction in a tensile test performed to measure the toughness value, where the unit is μm·mm.

The A value (toughness value) is measured in the following manner. First, the measurement target (pressure-sensitive adhesive composition or its cured product (in the case of the semi-curing type, primary cured product or secondary cured product)) is cut to prepare a film-type specimen. At this time, the film-type specimen is prepared in a form having a predetermined thickness with a width of 4cm or so and a length of 1cm or so.

The shape of such a specimen is exemplarily shown in Figure 3. Subsequently, the specimen is mounted on a tensile tester (e.g., TA equipment (Texture analyser plus)) and tensioned at a constant speed. At this time, the direction of the tension is parallel to the transverse direction of the specimen. For example, both ends of the specimen may be fixed to the tensile tester by 1 cm, and the specimen may be tensioned at a constant speed. At this time, the tension is performed at a tensile speed of about 1,000 mm/min. As exemplarily shown in Figure 2, a tensile curve is created with the tensile strength (gf) measured in the tensile process as the y-axis and the tensile distance (mm) as the x-axis, and the integral value (area of the graph) of the tensile curve at a tensile distance of 200 mm is designated as toughness, which is the A value in Equation 1 above.

Meanwhile, in Equation 1, S is the cross-sectional area of the specimen in the tensile direction in the tensile test for obtaining A, and is indicated by S in Figure 3. At this time, the unit of S is μm·mm. That is, when the tensile direction is the transverse direction of the specimen, S can be obtained by multiplying the longitudinal length (1cm) of the specimen by the thickness, where the longitudinal length uses a value in the unit of mm, and the thickness uses a value in the unit of μm.

In another example, the punching factor of the curable composition before or after curing may be about 25 gf/μm or more, 26 gf/μm or more, about 27 gf/μm or more, about 29 gf/μm or more, about 31 gf/μm or more, about 33 gf/μm or more, or about 35 gf/μm or more, or may also be about 98 gf/μm or less, about 96 gf/μm or less, about 94 gf/μm or less, about 92 gf/μm or less, about 90 gf/μm or less, about 88 gf/μm or less, about 86 gf/μm or less, about 84 gf/μm or less, about 82 gf/μm or less, about 80 gf/μm or less, about 78 gf/μm or less, about 76 gf/μm or less, about 74 gf /μm or less, about 72 gf/μm or less, about 70 gf/μm or less, about 68 gf/μm or less, 65 gf/μm or less, 60 gf/μm or less, 55 gf/μm or less, 50 gf/μm or less, about 45 gf/μm or less, about 43 gf/μm or less, about 41 gf/μm or less, about 39 gf/μm or less, or about 37 gf/μm or less or so.

By composing the curable composition to have such a punching factor, it may have excellent punching properties, prevent white turbidity and implement a low dielectric constant, while having the desired step embedding properties or

step absorptivity.

The polymer component included in the curable composition exhibiting such a punching factor may comprise, as monomer units, an alkyl acrylate unit having a straight or branched alkyl group, a hydroxy group-containing monomer unit and a nitrogen-containing monomer unit.

As described above, the monomer unit may mean a monomer that exists in a state where an oligomer or polymer is formed in the polymer component, or a monomer that is not polymerized and is included in the syrup component.

The alkyl group of the alkyl acrylate unit having a straight or branched alkyl group included in the polymer component may be an alkyl group having 4 to 20 carbon atoms. In another example, the number of carbon atoms of the alkyl group may be 16 or less, 12 or less, or 8 or less, which may be in a substituted or unsubstituted state.

As the alkyl acrylate, any one or two or more selected from the group consisting of, for example, n-butyl acrylate, t-butyl acrylate, sec-butyl acrylate, pentyl acrylate, 2-ethylbutyl acrylate, 2-ethylhexyl acrylate, n-octyl acrylate, isooctyl acrylate, lauryl acrylate and tetradecyl acrylate may be applied, without being limited thereto.

Such an alkyl acrylate may be included in the polymer component in a ratio of about 50 to 95 weight%. In another example, the ratio may be 52 weight% or more, 54 weight% or more, 56 weight% or more, 58 weight% or more, 60 weight% or more, 62 weight% or more, 64 weight% or more, 66 weight% or more, 68 weight% or more, 70 weight% or more, 72 weight% or more, 74 weight% or more, 76 weight% or more, 78 weight% or more, or 80 weight% or more, or may also be 93 weight% or less, 91 weight% or less, 89 weight% or less, 87 weight% or less, 85 weight% or less, 83 weight% or less, or 81 weight% or less or so.

The polymer component comprises a hydroxy group-containing monomer unit and a nitrogen-containing monomer unit together with the alkyl acrylate unit.

In the present application, the two monomer units are applied and their ratios are controlled as necessary, whereby proper punching properties and step embedding properties or step absorptivity may be simultaneously achieved, as well as the dielectric constant increase or the white turbidity occurrence due to polar monomers may be effectively prevented.

For example, the hydroxy group-containing monomer unit may be included so that the white turbidity is suppressed in high temperature and/or high humidity environments and the dielectric constant is not increased excessively, while maintaining the punching performance and handleability of the curable composition at an appropriate level.

For example, when the ratio of the hydroxy group-containing monomer unit is reduced, it is advantageous in terms of step absorptivity or step embedding properties and low dielectric constant, but there is a tendency for punching properties to be lowered and for white turbidity prevention ability to be lowered. Therefore, the ratio of the hydroxy group-containing monomer unit is maintained at an appropriate level and simultaneously the insufficient portion is supplemented with the nitrogen-containing monomer unit, whereby the curable composition satisfying all desired physical properties may be provided.

Accordingly, in one example, the total weight of the hydroxy group-containing monomer unit and the nitrogen-containing monomer unit may be controlled at a ratio of 8 to 20 parts by weight relative to 100 parts by weight of the alkyl acrylate unit. In another example, the ratio may be 9 parts by weight or more, 10 parts by weight or more, 11 parts by weight or more, or 12 parts by weight or more, or may also be 19 parts by weight or less, 18 parts by weight or less, 17 parts by weight or less, 16 parts by weight or less, 15 parts by weight or less. 14 parts by weight or less, or 13 parts by weight or less or so.

Also, in the polymer component, the nitrogen-containing monomer unit may also be present in a ratio of 2 to 10 parts by weight relative to 100 parts by weight of the alkyl acrylate unit. In another example, the ratio may be 3 parts by weight or more, 4 parts by weight or more, 5 parts by weight or more, 6 parts by weight or more, or 7 parts by weight or more, or may also be 9 parts by weight or less, 8 parts by weight or less, 7 parts by weight or less, or 6 parts by weight or less or so.

In addition, the weight ratio (B/A) of the hydroxy group-containing monomer unit (B) to the nitrogen-containing monomer unit (A) in the polymer component may be in a range of 0.1 to 5. In another example, the ratio (B/A) may be 0.2 parts by weight or more, 0.3 parts by weight or more, 0.4 parts by weight or more, 0.5 parts by weight or more, 0.6 parts by weight or more, 0.7 parts by weight or more, 0.8 parts by weight or more, 0.9 parts by weight or more, 1 part by weight or more, 1.1 parts by weight or more, 1.2 parts by weight or more, 1.3 parts by weight or more, 1.4 parts by weight or more, or 1.5 parts by weight or more, or may also be 4 parts by weight or less, 3 parts by weight or less, 2 parts by weight or less, 1.9 parts by weight or less, 1.8 parts by weight or less, 1.7 parts by weight or less, 1.6 parts by weight or less, 1.5 parts by weight or less, 1.4 parts by weight or less, 1.3 parts by weight or less, 1.2 parts by weight or less, 1.1 parts by weight or less, 1 part by weight or less, 0.9 parts by weight or less, 0.8 part by weight or less, or 0.7 part by weight or less or so.

Under such a ratio, the desired physical properties can be effectively achieved.

In another example, the hydroxy group-containing monomer unit and the nitrogen-containing monomer unit in the polymer component are included in a range of more than 8 parts by weight to less than 12 parts by weight based on 100 parts by weight of the total polymer component. In addition, the nitrogen-containing monomer unit is included in

a range of more than 2 parts by weight to 6 parts by weight or less based on 100 parts by weight of the polymer component.

Within such ranges, it is possible to provide a curable composition maintaining appropriate tensile strength and preventing white turbidity in high-temperature/high-humidity environments while ensuring step absorptivity or step embedding properties of the curable composition.

In one example, as the hydroxy group-containing monomer of the hydroxy group-containing monomer unit, a compound of Formula 1 below may be used.

[Formula 1]

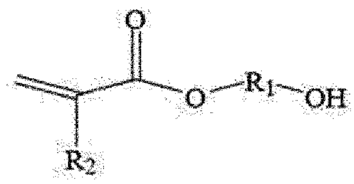

In Formula 1, $R_1$ is an alkylene group, and R2 is hydrogen or an alkyl group.

In one example, the alkylene group or alkyl group in Formula 1 may be an alkylene group or alkyl group having 1 to 20 carbon atoms, 1 to 16 carbon atoms, 1 to 12 carbon atoms, 1 to 8 carbon atoms, or 1 to 4 carbon atoms, where this alkylene group or alkyl group may be straight, branched or cyclic, which may be in a state optionally substituted by one or more substituents, or an unsubstituted state.

In one example, R2 in Formula 1 may be hydrogen or a methyl group.

The compound of Formula 1 may be exemplified by, for example, hydroxymethyl (meth)acrylate, 2-hydroxyethyl (meth)acrylate, 3-hydroxypropyl (meth)acrylate, 4-hydroxybutyl (meth)acrylate, 5-hydroxypentyl (meth)acrylate or 6-hydroxyhexyl (meth)acrylate, and the like, but is not limited thereto.

When the compound of Formula 1 is used as the hydroxy group-containing monomer, it may be more advantageous to simultaneously secure excellent tensile strength, prevention of reduction in the effect of suppressing white turbidity in high-temperature/high-humidity environments, low dielectric constant, and excellent step absorption performance.

As the nitrogen-containing monomer of the nitrogen-containing monomer unit, one or more selected from the group consisting of (meth)acrylamide, N-alkyl (meth)acrylamide, N,N-dialkyl (meth)acrylamide, 1-aminobut-3-en-2-one, 1-(dimethylamino)but-3-en-2-one, 1-(dimethylamino)-3-methylbut-3-en-2-one, 5-aminopent-1-en-3-one, 5-(dimethylamino)pent-1-en-3-one, 5-(dimethylamino)-2-methylpent-1-en-3-one, 5-amino-2-methylpent-1-en-3-one and 5-amino-2-methylhex-1-en-3-one may be used, without being limited thereto. Specifically, the alkyl group used while defining the type of the nitrogen-containing monomer may be an alkyl group having 1 to 20 carbon atoms, 1 to 16 carbon atoms, 1 to 12 carbon atoms, 1 to 8 carbon atoms, or 1 to 4 carbon atoms, where this alkylene group or alkyl group may be straight, branched or cyclic, which may be in a state optionally substituted by one or more substituents, or an unsubstituted state.

In terms of securing an appropriate effect, it may be appropriate to use acrylamide, without being limited thereto.

The polymer component may comprise, as an additional monomer unit, an alkyl methacrylate unit, and in one example, it may further comprise an alkyl methacrylate unit having a straight or branched alkyl group.

The alkyl group of the alkyl methacrylate unit having a straight or branched alkyl group may be an alkyl group having 4 to 20 carbon atoms. In another example, the number of carbon atoms of the alkyl group may be 16 or less, 12 or less, or 8 or less, which may be in a substituted or unsubstituted state.

As the alkyl methacrylate, for example, one or more selected from the group consisting of n-butyl methacrylate, t-butyl methacrylate, sec-butyl methacrylate, pentyl methacrylate, 2-ethylbutyl methacrylate, 2-ethylhexyl methacrylate, n-octyl methacrylate, isooctyl methacrylate, lauryl methacrylate and tetradecyl methacrylate may be applied, without being limited thereto.

Such an alkyl methacrylate unit may be included in the polymer component in a ratio of about 1 to 20 parts by weight relative to 100 parts by weight of the alkyl acrylate unit. In another example, the ratio may be 3 parts by weight or more, 5 parts by weight or more, 7 parts by weight or more, 7.5 parts by weight or more, 8 parts by weight or more, or 8.5 parts by weight or more or so, or may also be 18 parts by weight or less, 16 parts by weight or less, 14 parts by weight or less, 12 parts by weight or less, 10 parts by weight or less, or 9 parts by weight or less or so.

The polymer component may comprise, as an additional monomer unit, a unit of a compound of Formula 2 below as a monomer unit.

[Formula 2]

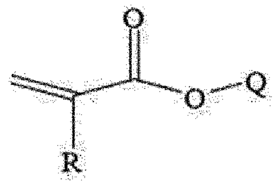

In Formula 2, R is hydrogen or an alkyl group, and Q is a monovalent substituent having a non-aromatic ring structure with 3 to 20 carbon atoms.

In Formula 2, the alkyl group may be exemplified by a straight, branched or cyclic substituted or unsubstituted alkyl group having 1 to 20 carbon atoms, 1 to 16 carbon atoms, 1 to 12 carbon atoms, 1 to 8 carbon atoms, or 1 to 4 carbon atoms.

Meanwhile, Q is a monovalent substituent having a non-aromatic ring structure with 3 to 20 carbon atoms, which may be, for example, a monovalent substituent derived from an aliphatic saturated or unsaturated hydrocarbon cyclic compound. The cyclic compound may have a monocyclic structure, or may also have a polycyclic structure such as a condensed or spiro type. In another example, the number of carbon atoms in the ring structure may be 6 or more, or 8 or more, or may be 18 or less, 16 or less, 14 or less, or 12 or less. Such a substituent may be exemplified by, for example, an isobornyl group, a cyclohexyl group, a norbornanyl group, a norbornenyl group, a dicyclopentadienyl group, an ethynylcyclohexane group, an ethynylcyclohexene group or an ethynyldecahydronaphthalene group, and the like, without being limited thereto.

The unit of the compound of Formula 2 may be included in an amount of 0.1 to 10 parts by weight relative to 100 parts by weight of an alkyl acrylate unit having a straight or branched alkyl group. In another example, this ratio may be 0.5 parts by weight or more, 1 part by weight or more, 1.5 parts by weight or more, 2 parts by weight or more, 2.5 parts by weight or more, 3 parts by weight or more, or 3.5 parts by weight or more, or may also be 9 parts by weight or less, 8 parts by weight or less, 7 parts by weight or less, 6 parts by weight or less, 5 parts by weight or less, or 4 parts by weight or less or so.

As the polymer component further comprises the above units, it is possible to more stably provide a curable composition having the desired physical properties.

As one example, the polymer component may have a weight average molecular weight (Mw) in a range of about 50,000 to 120,000. As another example, the polymer component may have a weight average molecular weight (Mw) of about 60,000 or more, 70,000 or more, or about 80,000 or more, and it may be about 115,000 or less, 110,000 or less, or about 105,000 or less.

The weight average molecular weight (g/mol) may mean a value converted to polystyrene measured in the manner described in these examples by GPC (gel permeation chromatography), and unless otherwise specified, the molecular weight of any polymer may mean the weight average molecular weight of the polymer.

By controlling the weight average molecular weight of the polymer component within the above range, it is possible to effectively secure an appropriate crosslinking effect, tensile performance and step absorptivity, and the like.

The polymer component may comprise a polymer (i.e., a polymer or an oligomer as a component in the form that two or more monomers are polymerized) in a ratio within the range of 60 to 80 weight%.

The curable composition may comprise a curing agent together with the polymer component. In order to secure a desired crosslinked structure, two or more curing agents may be used as the curing agent.

For example, as the curing agent, two or more selected from the group consisting of a polyfunctional acrylate having a molar mass of 500 g/mol or less, a polyfunctional isocyanate compound and a polyfunctional urethane acrylate may be applied.

Here, the term polyfunctional acrylate means a compound containing two or more acrylate functional groups such as a (meth)acryloyl group and/or a (meth)acryloyloxy group. At this time, the number of the acrylate functional groups may be present in, for example, a number within a range of 2 to 10, 2 to 9, 2 to 8, 2 to 7, 2 to 6, 2 to 5, 2 to 4 or 2 to 3. For an appropriate crosslinking structure, a bifunctional acrylate may also be applied as the polyfunctional acrylate.

As the acrylate compound, for example, one or two or more selected from the group consisting of a bifunctional type such as 1,4-butanediol di(meth)acrylate, 1,6-hexanediol di(meth)acrylate, neopentyl glycol di(meth)acrylate, hydroxypivalic acid neopentyl glycol di(meth)acrylate, dicyclopentanyl di(meth)acrylate, caprolactone-modified dicyclopentenyl di(meth)acrylate, ethylene oxide-modified phosphoric acid di(meth)acrylate, di(meth)acryl oxyethyl isocyanurate, allylated cyclohexyl di(meth)acrylate, tricyclodecanedimethanol di(meth)acrylate, dimethyloldicyclopentane di(meth)acrylate, ethylene oxide modified hexahydrophthalic acid di(meth)acrylate, neopentyl glycol modified trimethylolpropane di(meth)acrylate, adamantane di(meth)acrylate and 9,9-bis[4-(2-acryloyloxyethoxy)phenyl]fluorene; a trifunctional type

such as trimethylolpropane tri(meth)acrylate, dipentaerythritol tri(meth)acrylate, propionic acid modified dipentaerythritol tri(meth)acrylate, pentaerythritol tri(meth)acrylate, propylene oxide modified trimethylolpropane tri(meth)acrylate and tris(meth)acryloxyethyl isocyanurate; a tetrafunctional type such as diglycerin tetra(meth)acrylate and pentaerythritol tetra(meth)acrylate; a pentafunctional type such as propionic acid modified dipentaerythritol penta(meth)acrylate; and a hexafunctional type such as dipentaerythritol hexa(meth)acrylate, caprolactone-modified dipentaerythritol hexa(meth)acrylate, and the like may be used.

In one example, a component having a molecular weight of about 500 g/mol or less may be applied as the polyfunctional acrylate. In another example, the molecular weight may be about 450 g/mol or less, about 400 g/mol or less, about 350 g/mol or less, about 300 g/mol or less, or about 250 g/mol or less, or may also be about 50 g/mol or more, about 55 g/mol or more, about 60 g/mol or more, about 65 g/mol or more, about 70 g/mol or more, about 75 g/mol or more, about 80 g/mol or more, about 85 g/mol or more, about 90 g/mol or more, about 95 g/mol or more, about 100 g/mol or more, about 110 g/mol or more, about 120 g/mol or more, about 130 g/mol or more, about 140 g/mol or more, about 150 g /mol or more, about 160 g/mol or more, about 170 g/mol or more, about 180 g/mol or more, about 190 g/mol or more, or about 200 g/mol or more or so.

In the present application, an aliphatic acyclic polyfunctional acrylate may be applied as the polyfunctional acrylate having the above molecular weight, without being limited thereto.

The polyfunctional isocyanate compound that can be applied as the curing agent is a compound containing two or more isocyanate functional groups, where as an example thereof, a diisocyante such as tolylene diisocyanate, xylene diisocyanate, diphenylmethane diisocyanate, hexamethylene diisocyanate, isophorone diisocyanate, tetramethylxylene diisocyanate or naphthalene diisocyanate, or a reaction product of one or more of the above diisocyanates and a polyol (e.g., trimethylol propane), and the like may be used.

As the urethane acrylate, a reaction product of a polyol, a polyfunctional isocyanate and a hydroxy group-containing (meth)acrylate or a reaction product of a polyfunctional isocyanate and a hydroxy group-containing (meth)acrylate may be applied. Therefore, the urethane acrylate may comprise a polyol unit, a polyfunctional isocyanate unit and a hydroxy group-containing (meth)acrylate unit, or may comprise a polyfunctional isocyanate unit and a hydroxy group-containing (meth)acrylate unit.

Here, a diol may be applied as the polyol, and as the diol, any one or two or more selected from a low molecular weight diol, a diol having a polyene skeleton, a diol having a polyester skeleton, a diol having a polyether skeleton and a diol having a polycarbonate skeleton may be applied. The low molecular weight diol may be exemplified by ethylene glycol, propylene glycol, cyclohexanedimethanol, neopentyl glycol, 3-methyl-1,5-pentanediol and/or 1,6-hexanediol, and the like; the diol having a polyene skeleton may be exemplified by a diol having a polybutadiene skeleton, a diol having a polyisoprene skeleton, a diol having a hydrogenated polybutadiene skeleton and/or a diol having a hydrogenated polyisoprene skeleton, and the like; and the diol having a polyester skeleton may exemplified by an esterification product of a diol component such as the low molecular weight diol or polycaprolactone diol and an acid component such as dicarboxylic acid or an anhydride thereof, and the like.

Here, as the dicarboxylic acid or anhydride thereof, adipic acid, succinic acid, phthalic acid, tetrahydrophthalic acid, hexahydrophthalic acid and/or terephthalic acid and anhydrides thereof, and the like may be exemplified. In addition, the polyether diol may be exemplified by polyethylene glycol and/or polypropylene glycol, and the like, and the polycarbonate diol may be exemplified by a reaction product of the low molecular weight diol or/and a bisphenol such as bisphenol A with a carbonic acid dialkyl ester such as ethylene carbonate and dibutyl carbonate ester, and the like.

As the diol having a polyene skeleton, a diol having a hydrogenated polydiene skeleton may be applied, and an example thereof may include a compound having a diol at the polymer terminal of a monomer such as isoprene, 1,3-butadiene and 1,3-pentadiene, and a compound having a diol at the terminal of the polymer of these monomers.

As the polyfunctional isocyanate, for example, an aliphatic diisocyanate such as hexamethylene diisocyanate, lysine methyl ester diisocyanate, 2,4,4-trimethylhexamethylene diisocyanate and/or dimer acid diisocyanate, an alicyclic diisocyanate such as isophorone diisocyanate, 4,4'-methylenebis(cyclohexyl isocyanate) and/or diisocyanate dimethylcyclohexane, an aromatic diisocyanate such as tolylene diisocyanate, xylene diisocyanate and/or diphenylmethane-4,4'-diisocyanate, and the like may be used. Furthermore, as the polyfunctional isocyanate having three or more isocyanate groups, a hexamethylene diisocyanate trimer and isophorone diisocyanate trimer, and the like may also be applied.

As the hydroxy group-containing (meth)acrylate, a hydroxyalkyl (meth)acrylate such as 2-hydroxyethyl (meth)acrylate, 2-hydroxypropyl (meth)acrylate, 4-hydroxybutyl (meth)acrylate, hydroxypentyl (meth)acrylate and/or hydroxyhexyl (meth)acrylate and hydroxyoctyl (meth)acrylate, and/or a compound having a hydroxy group and two or more (meth)acryloyl groups such as mono or di(meth)acrylate of trimethylolpropane, mono, di or tri(meth)acrylate of pentaerythritol, mono, di or tri(meth)acrylate of ditrimethylolpropane and/or mono, di, tri, tetra or penta(meth)acrylate of dipentaerythritol, and the like may be applied.

A method for preparing a desired urethane acrylate by applying the above components is known. For example, the urethane acrylate may be prepared by a method of reacting a polyol and a polyfunctional isocyanate according to a known method to prepare an isocyanate group-containing compound and reacting the same with a hydroxy group-containing (meth)acrylate, or a method of reacting a polyol, a polyfunctional isocyanate and a hydroxy group-containing (meth)acrylate simultaneously or reacting a polyfunctional isocyanate and a hydroxy group-containing (meth)acrylate.

In one example, one having a number average molecular weight (Mn) in a range of 2,000 to 50,000 g/mol may be used as the urethane acrylate, and a bifunctional urethane acrylate, that is, one comprising two functional groups selected from a (meth)acryloyl group and a (meth)acryloyloxy group may be used. The number average molecular weight is a converted value of standard polystyrene measured by a GPC (gel permeation chromatograph) method.

In one example, as the urethane acrylate, a bifunctional aliphatic polyfunctional urethane acrylate may be used, and for example, a product marketed as SUO 1020 (manufactured by SHIN-A T&C), SUO 1200 (manufactured by SHIN-A T&C), SUO 4120 (manufactured by SHIN-A T&C), SUO M2000 (manufactured by SHIN-A T&C) or SUO 2150 (manufactured by SHIN-A T&C), and the like may be used.

In the present application, two suitable curing agents may be selected from the curing agents as above and used. In one example, in order to properly achieve the desired chemical crosslinking and physical entanglement, an isocyanate compound may not be applied as the curing agent. Therefore, in one example, the compound having an isocyanate group may not exist in the curable composition of the present application. Since it is a compound having an isocyanate group, the case where it is applied to the production of the urethane acrylate and the isocyanate group has already disappeared is excluded.

The curing agent may be used in an amount of 0.01 to 10 parts by weight relative to 100 parts by weight of the polymer component. In another example, the ratio may be 0.05 parts by weight or more, 0.1 parts by weight or more, 0.15 parts by weight or more, or 0.2 parts by weight or more or so, or may also be 9 parts by weight or less, 8 parts by weight or less, 7 parts by weight or less, 6 parts by weight or less, 5 parts by weight or less, 4 parts by weight or less, 3 parts by weight or less, 2 parts by weight or less, 1 part by weight or less, 0.9 parts by weight or less, 0.8 parts by weight or less, 0.7 parts by weight or less, 0.6 parts by weight or less, 0.5 parts by weight or less, 0.4 parts by weight less, or 0.3 parts by weight or less or so.

When two or more curing agents are applied, the ratio of each curing agent as applied may be adjusted according to the purpose.

For example, when using a polyfunctional acrylate having a molar mass of 500 g/mol or less and a polyfunctional urethane acrylate as the curing agent, the polyfunctional urethane acrylate is included in a ratio of 0.01 parts by weight to 10 parts by weight relative to 100 parts by weight of the polymer component, where the weight ratio (C/D) of the polyfunctional urethane acrylate (C) to the polyfunctional acrylate (D) having a molar mass of 500 g/mol or less may be in the range of 5 to 50.

In another example, the polyfunctional urethane acrylate may be included in an amount of 0.05 parts by weight or more, 0.1 parts by weight or more, 0.15 parts by weight or more, or 0.2 parts by weight or more, or may also be included in an amount of 9 parts by weight or less, 8 parts by weight or less, 7 parts by weight or less, 6 parts by weight or less, 5 parts by weight or less, 4 parts by weight or less, 3 parts by weight or less, 2 parts by weight or less, 1 part by weight or less, 0.9 parts by weight or less, 0.8 parts by weight or less, 0.7 parts by weight or less, 0.6 parts by weight or less, 0.5 weight part or less, 0.4 weight part or less, or 0.3 weight part or less or so, relative to 100 parts by weight of the polymer component.

Also, in another example, the weight ratio (C/D) may be 10 or more, 15 or more, or 18 or more, or may also be 45 or less, 40 or less, 35 or less, 30 or less, 25 or less, or 22 or less or so.

Accordingly, the desired crosslinked structure may be ensured.

The curable composition may further comprise necessary components in addition to the above components. For example, the curable composition may further comprise a radical initiator, such as a photo radical initiator.

As the radical initiator, for example, benzoin, benzoin methyl ether, benzoin ethyl ether, benzoin isopropyl ether, benzoin n-butyl ether, benzoin isobutyl ether, acetophenone, dimethylaminoacetophenone, 2,2-dimethoxy-2-phenylacetophenone, 2,2-diethoxy-2-phenylacetophenone, 2-hydroxy-2-methyl-1-phenylpropan-1-one, 1-hydroxycyclohexylphenyl ketone, 2-methyl-1-[4-(methylthio)phenyl]-2-morpholino-propan-1-one, 4-(2-hydroxyethoxy)phenyl-2-(hydroxy-2-propyl) ketone, benzophenone, p-phenylbenzophenone, 4,4'-diethylaminobenzophenone, dichlorobenzophenone, 2-methylanthraquinone, 2-ethylanthraquinone, 2-t-butylanthraquinone, 2-aminoanthraquinone, 2-methylthioxanthone, 2-ethylthioxanthone, 2-chlorothioxanthone, 2,4-dimethylthioxanthone, 2,4-diethylthioxanthone, benzyldimethylketal, acetophenone dimethylketal, p-dimethylaminobenzoic acid ester, oligo[2-hydroxy-2-methyl-1-[4-(1-methylvinyl)phenyl]propanone] or 2,4,6-trimethylbenzoyl-diphenyl-phosphineoxide, and the like may be used, without being limited thereto.

For example, the radical initiator may be included in an appropriate ratio in the curable composition, which may be included, for example, in a ratio of approximately 0.1 to 3 parts by weight relative to 100 parts by weight of the polymer component.

The curable composition may further comprise a silane coupling agent. The silane coupling agent may improve adhesiveness and adhesion stability of the pressure-sensitive adhesive, thereby improving heat resistance and moisture resistance, and may also act to improve adhesion reliability even when left for a long time under severe conditions. As the silane coupling agent, for example, gamma-glycidoxypropyl triethoxy silane, gamma-glycidoxypropyl trimethoxy

silane, gamma-glycidoxypropyl methyldiethoxy silane, gamma-glycidoxypropyl triethoxy silane, 3-mercaptopropyl trimethoxy silane, vinyltrimethoxysilane, vinyltriethoxysilane, gamma-methacryloxypropyl trimethoxysilane, gamma-methacryloxypropyl triethoxysilane, gamma-aminopropyl trimethoxysilane, gamma-aminopropyl triethoxysilane, 3-isocyanatopropyl triethoxysilane, gamma-acetoacetatepropyl trimethoxysilane, gamma-acetoacetatepropyl triethoxysilane, beta-cyanoacetyl trimethoxysilane, beta-cyanoacetyl triethoxysilane, acetoxyaceto trimethoxysilane, and the like may be used, and one, or a mixture of two or more of the foregoing may be used. It is preferable to use a silane-based coupling agent having an acetoacetate group or a beta-cyanoacetyl group, without being limited thereto.

The silane coupling agent may be included in the curable composition in an appropriate ratio, which may be included, for example, in a ratio of about 0.01 parts by weight to about 5 parts by weight or so relative to 100 parts by weight of the polymer component.

The curable composition may further comprise an antioxidant if desired. As the antioxidant, for example, a hindered phenol-based compound, a sulfur-based antioxidant, a phenyl acetic acid-based antioxidant or a phosphorus-based antioxidant, and the like may be applied, without being limited thereto.

Such an antioxidant may be included in an amount of about 0.01 parts by weight to about 5 parts by weight or about 0.01 parts by weight to about 1 part by weight relative to 100 parts by weight of the polymer component in the curable composition.

The curable composition may also comprise an antistatic agent. As the antistatic agent, an ionic compound is usually applied. As the ionic compound, for example, a metal salt or an organic salt may be used.

Such an antistatic agent may be included in an amount of about 0.01 to 10 parts by weight relative to 100 parts by weight of the polymer component in the curable composition, where an appropriate ratio may be applied in consideration of desired conductivity or the like.

As one example, the curable composition according to the present application may comprise a photoinitiator. As long as the photoinitiator is capable of initiating the polymerization reaction of the curable composition through light irradiation or the like, any one may be used. For example, it may include alpha-hydroxyketone-based compounds (e.g., IRGACURE 184, IRGACURE 500, IRGACURE 2959, DAROCUR 1173; manufactured by Ciba Specialty Chemicals); phenylglyoxylate-based compounds (e.g., IRGACURE 754, DAROCUR MBF; manufactured by Ciba Specialty Chemicals); benzyl dimethyl ketal-based compounds (e.g., IRGACURE 651; manufactured by Ciba Specialty Chemicals); a-aminoketone-based compounds (e.g., IRGACURE 369, IRGACURE 907, IRGACURE 1300; manufactured by Ciba Specialty Chemicals); monoacylphosphine-based compounds (MAPO) (e.g., DAROCUR TPO; manufactured by Ciba Specialty Chemicals); bisacylphosphene-based compounds (BAPO) (e.g., IRGACURE 819, IRGACURE 819DW; manufactured by Ciba Specialty Chemicals); phosphine oxide-based compounds (e.g., IRGACURE 2100; manufactured by Ciba Specialty Chemicals); metallocene-based compounds (e.g., IRGACURE 784; manufactured by Ciba Specialty Chemicals); iodonium salts (e.g., IRGACURE 250; manufactured by Ciba Specialty Chemicals); and mixtures of one or more of the foregoing (e.g., DAROCUR 4265, IRGACURE 2022, IRGACURE 1300, IRGACURE 2005, IRGACURE 2010, IRGACURE 2020; manufactured by Ciba Specialty Chemicals), and one or two or more of the foregoing may be used, without being limited thereto. In an embodiment of the present application, the pressure-sensitive adhesive composition may comprise, as the photoinitiator, a photoinitiator having an absorption wavelength band of 400nm or more. The present application can implement excellent curing properties by adjusting the absorption wavelength band of the photoinitiator.

The photoinitiator may be included in an amount of 2 parts by weight or less relative to 100 parts by weight of the polymer component. As another example, it may be about 1 part by weight or less, or about 0.5 parts by weight or less, and it may be included in about 0.001 parts by weight or more, about 0.005 parts by weight or more, or about 0.01 parts by weight or more. When the photoinitiator is included in the curable composition within the above range, curing efficiency may be increased.

As one example, the curable composition may further comprise a post-curing photoinitiator (hereinafter, may be referred to as a 'second photoinitiator') absorbing a wavelength region in a range of 260nm to 330nm in addition to the photoinitiator (hereinafter, may be referred to as a 'first photoinitiator') absorbing a wavelength band of 400nm or more as described above.

For example, as the second photoinitiator, a compound of Formula 3 below may be used. Such a compound may be included in the polymer component as a monomer unit, or may be included in the curable composition as a separate component.

[Formula 3]

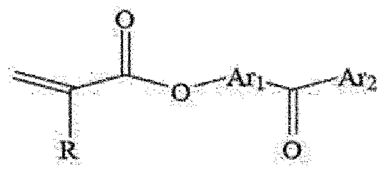

In Formula 3, R is hydrogen or an alkyl group, An is an arylene group, and Ar2 is an aryl group.

In Formula 3, the alkyl group may be exemplified by a straight, branched or cyclic substituted or unsubstituted alkyl group having 1 to 20 carbon atoms, 1 to 16 carbon atoms, 1 to 12 carbon atoms, 1 to 8 carbon atoms or 1 to 4 carbon atoms.

In Formula 3, the arylene group or aryl group may be an arylene or aryl group having 6 to 30 carbon atoms, 6 to 26 carbon atoms, 6 to 22 carbon atoms, 6 to 18 carbon atoms, 6 to 14 carbon atoms, 6 to 12 carbon atoms, or may be a phenylene group or a phenyl group, where such an arylene group or aryl group may be in a substituted state or may also be in an unsubstituted state.

An example of the compound of Formula 3 may be exemplified by benzoylphenyl methacrylate or the like, but is not limited thereto.

The second photoinitiator may be included in a range of 0.001 to 1 part by weight relative to 100 parts by weight of the polymer component.

Under the above ratio, it is possible to provide a curable composition having desired physical properties while achieving appropriate post-curing.

As one example, the curable composition or its cured product (in the case of the semi-curing type, primary cured product or secondary cured product (post-cured product)) may have a maximum tensile strength in a range of greater than 250 gf to 600 gf or less. As another example, it may be about 260 gf or more, or about 270 gf or more, and may be about 600 gf or less, 580 gf or less, 560 gf or less, or about 540 gf or less.

The maximum tensile strength means a tensile strength when the specimen is fractured during the tensile test process, where the method of performing the tensile test is similar to the case of obtaining the A value of Equation 1 above.

Specifically, first, as in the measurement of the A value (toughness value), the measurement target (pressure-sensitive adhesive composition or its cured product (in the case of the semi-curing type, primary cured product or secondary cured product)) is cut to prepare a film-type specimen. At this time, the film-type specimen should have a width of 4cm or so, a length of 1cm or so, and a thickness of about 600μm or so.

Subsequently, the specimen is mounted on a tensile tester (e.g., TA equipment (Texture analyser plus)) and tensioned at a constant speed. At this time, the direction of the tension is parallel to the transverse direction of the specimen. For example, both ends of the specimen may be fixed to the tensile tester by 1 cm, and the specimen may be tensioned at a constant speed. At this time, the tension is performed at a tensile speed of about 1,000 mm/min.

While performing the tension at a speed of about 1,000 mm/min in such a manner, the tensile strength when the specimen is broken can be set as the maximum tensile strength.

By having such a maximum tensile strength, it is possible to secure appropriate punching performance, handling properties and step absorption performance.

As one example, the curable composition or its cured product (in the case of the semi-curing type, primary cured product or secondary cured product (post-cured product)) may have a dielectric constant of 4.0 or less at a frequency of 100 kHz. In another example, it may be about 3.9 or less, about 3.8 or less, or about 3.7 or less, and may be about 2.0 or more, 2.1 or more, 2.2 or more, 2.3 or more, 2.4 or more, or about 2.5 or more, 2.6 or more, 2.7 or more, 2.8 or more, 2.9 or more, 3 or more, 3.1 or more, 3.2 or more, 3.3 or more, 3.4 or more, or 3.5 or more or so.

The pressure-sensitive adhesive composition according to the present application satisfies Equation 1 as described above, and comprises a hydroxy group-containing monomer unit and a nitrogen-containing monomer unit in a predetermined content ratio in the polymer component, where it is advantageous for the cured product formed from the pressure-sensitive adhesive composition comprising the same to ensure a dielectric constant of 4.0 or less at a frequency of 100 kHz. Therefore, when applied to an optical member such as a touch panel, it is possible to impart excellent touch sensitivity.

The present application also relates to a pressure-sensitive adhesive film. Figure 1 is a schematic diagram illustratively showing a pressure-sensitive adhesive film of the present application. As shown in Figure 1, the pressure-sensitive adhesive film according to the present application may comprise a base material (20) and a cured product (10) of the pressure-sensitive adhesive composition formed on the base material. The cured product may be a primary cured product or a secondary cured product. As the pressure-sensitive adhesive composition, the above-described pressure-sensitive adhesive composition may be used. Therefore, the pressure-sensitive adhesive film comprising the cured

product formed from the pressure-sensitive adhesive composition may have excellent tensile strength, prevention of reduction in the effect of suppressing white turbidity in high temperature/high humidity environments, low dielectric constant, and excellent step absorption performance.

As one example, the base material is not particularly limited and a known base material may be used as long as the pressure-sensitive adhesive composition can be applied thereto. For example, a PET film subjected to a release treatment can be used.

The present application also relates to a display device comprising the curable composition or a cured product (pressure-sensitive adhesive, primary cured product or secondary cured product in the case of the semi-curing type) thereof. In one example, the display device may be a display device for vehicles. The curable composition or its cured product (pressure-sensitive adhesive) may be used as an OCA or OCR in the display device. Other constitutions of the display device or the application method of the OCA or OCR are not particularly limited, which may be applied in a known manner.

## Advantageous Effects

The present application may provide a curable composition having excellent step absorptivity, and step embedding and punching properties, preventing white turbidity, and being capable of implementing a low dielectric constant, and a use thereof.

Even if such a curable composition according to the present application is applied to a stepped substrate or the like, it may prevent lifting or delayed bubbles from being generated in the step portion, and simultaneously, it may maintain the effect of suppressing white turbidity in high temperature/high humidity environments, have a low dielectric constant and improve the punching performance and handling properties. In addition, the pressure-sensitive adhesive film comprising the cured product formed from the curable composition may have excellent tensile strength.

## Description of Drawings

Figure 1 is a schematic diagram illustratively showing a pressure-sensitive adhesive film of the present application.

Figure 2 is a view showing tensile strength (force) versus tensile distance (distance) as a graph.

Figure 3 is a schematic diagram illustratively showing a cured product of the pressure-sensitive adhesive composition.

## Mode for Invention

Hereinafter, the present application will be described in detail through examples, but the scope of the present application is not limited by the following examples.

### 1. Punching factor

The punching factor (F) (unit: gf/μm) value of the pressure-sensitive adhesive composition is obtained according to the following equation 1.

[Equation 1]

$$F = 10 \times (A/S)$$

In Equation 1, F is the punching factor (unit: gf/μm).

In Equation 1, A is the toughness value (toughness, unit: gf·mm) of the cured product (pressure-sensitive adhesive) of the pressure-sensitive adhesive composition, which is measured in the following manner.

First, the pressure-sensitive adhesive is cut into the form of a film to prepare a specimen. At this time, the film-type specimen is prepared by cutting the pressure-sensitive adhesive into the form of a film having a predetermined thickness with a width of 4 cm and a length of 1 cm. The film-type specimen is fixed in a tensile tester (TA equipment (Texture analyzer plus)) so that the transverse direction becomes the tensile direction, where both ends of the specimen in the transverse direction are fixed by 1 cm. Thereafter, the specimen is tensioned at a speed of about 1,000 mm/min or so until the tensile distance becomes 200 mm. The toughness value can be obtained by integrating the graph created

with the tensile strength (gf) in the tensile process as the y-axis and the tensile distance (mm) as the x-axis.

In Equation 1, S is the area of the surface (side) of the specimen in the tensile direction, and the unit is μm·mm. That is, the thickness of the side of the specimen is applied as a unit of μm, and the length (1 cm) in the longitudinal direction is converted into mm, whereby the S can be obtained.

## 2. Molecular weight evaluation

The weight average molecular weight (Mw) of the polymer component was measured using GPC (gel permeation chromatograph) under the following conditions, and the measurement results were converted using the standard polystyrene of Agilent system for manufacturing a calibration curve.

<Measurement conditions>

Meter: Agilent GPC (Agilent 1200 series, U.S.)

Column: PLGel-M, PLGel-L series connection

Column temperature: 35°C

Eluent: Tetrahydrofuran (THF)

Flow rate: 1.0 mL/min

Concentration: ~ 1 mg/mL (100 μL injection)

## 3. White turbidity evaluation

A glass substrate having a thickness of 1.1 mm and a glass substrate having a thickness of 0.55 mm were attached using each pressure-sensitive adhesive of Examples or Comparative Examples.

Subsequently, the laminate was left in an autoclave under conditions of 40°C and 4 bar for 10 minutes, and then, the pressure-sensitive adhesive was irradiated with ultraviolet rays at a light quantity of 3J using a metal halide lamp, and post-cured.

Thereafter, the pressure-sensitive adhesive attached to the glass was stored under conditions of 85°C and 85% relative humidity for 200 hours, and left at room temperature for 1 hour, and then it was observed whether or not white spots (or white turbidity) were observed. Table 1 below is the evaluation criteria for white turbidity performance.

[Table 1]

| Grade | White turbidity |
|---|---|
| O | No abnormality |
| X | White turbidity occurrence |

## 4. Step absorption evaluation

The pressure-sensitive adhesive films of Examples or Comparative Examples were each cut to have a width of 8.0cm and a length of 14.0cm, thereby preparing a specimen. Thereafter, on a glass substrate having a bezel part and a screen part with a printing step of 15μm in height, the primary lamination was performed with a pressure of 1.5 bar after one side of the release film was removed from the specimen, and after the release film on the other side of the specimen was removed, a glass substrate with a thickness of 0.55 mm was laminated thereon and the secondary lamination was performed with a pressure of 1.5 bar.

Subsequently, it was left in an autoclave under conditions of 40°C and 4 bar for 10 minutes, and then the step absorption performance was evaluated by checking the number of bubbles generated as the step difference was not sufficiently overcome at the four vertex parts of the bezel part and the number of bubbles present in the corner parts of the bezel part. Table 2 below is the evaluation criteria for step absorption.

[Table 2]

| Grade | Vertex bubbles | Corner bubbles |
|---|---|---|
| ◎ | 1-2 | Less than 10 |
| O | 3-4 | Less than 10 |
| Δ | 4 | 10 or more |
| X | 4 | 10 or more and line form |

## 5. Tensile evaluation

The pressure-sensitive adhesive layers prepared in Examples or Comparative Examples were each laminated by 6 sheets to have a thickness of 600μm or so, and then cut to have a width of 4.0cm or so and a length of 1.0cm or so, thereby preparing a specimen. Thereafter, both ends of the specimen were fixed by 1 cm in the TA equipment (Texture analyser plus) so that the transverse direction became the tensile direction, and the tensile test was performed by tensioning the specimen to 450 mm at a speed of about 1,000 mm/min. Thereafter, by evaluating the tensile strength, the tensile performance was evaluated based on the criteria shown in Table 3 according to the maximum tensile strength (unit: gf).

[Table 3]

| Grade | Maximum tensile strength |
|---|---|
| ◎ | More than 550 |
| O | More than 410 to 550 or less |
| Δ | More than 250 to 410 or less |
| X | 250 or less |

In the above evaluation criteria, the maximum tensile strength is a tensile strength when the specimen is fractured, where the unit is gf.

## 6. Dielectric constant evaluation

The pressure-sensitive adhesive films prepared in Examples or Comparative Examples were each cut to have a width of 2mm and a length of 5mm, and the pressure-sensitive adhesive layer was irradiated with ultraviolet rays at a light quantity of 3J using a metal halide lamp and post-cured. Thereafter, the dielectric constant was measured using a dielectric constant measuring device (Agilent, E4980A LCR meter + 16451B dielectric test fixture device).

## Example 1

### Preparation of polymer component

2-ethylhexyl acrylate (EHA), 2-hydroxyethyl acrylate (HEA), 2-ethylhexyl methacrylate (EHMA), acrylamide (AAm) and isobornyl acrylate (IBoA) were introduced into a 2L reactor in which nitrogen gas was refluxed and a cooling device was installed to facilitate temperature control in a ratio of 80:6:7:4:3 parts by weight (EHA: HEA: EHMA: AAm: IBoA) as shown in Table 4 below to form a monomer mixture. Subsequently, nitrogen gas was purged for 1 hour for removing oxygen, and AIBN (azobisisobutyronitrile) diluted to a concentration of 50 weight% in ethyl acetate was introduced thereto in an amount of about 0.03 parts by weight relative to 100 parts by weight of the monomer mixture in a state where the temperature was raised to about 67°C, and then reacted for 8 hours to prepare a syrup-type polymer component having a weight average molecular weight (Mw) of about 109,000 or so.

### Preparation of pressure-sensitive adhesive composition and pressure-sensitive adhesive film

With respect to 100 parts by weight of the prepared polymer component, additional components were blended as shown in Table 5 below to prepare a curable composition (pressure-sensitive adhesive composition). Subsequently,

the curable composition was applied to the release-treated surface of the release-treated release PET (poly(ethylene terephthalate)) film, and the release-treated surface of an additional release PET film was laminated on the coating layer, and then was irradiated (1J) with ultraviolet rays by means of a metal halide lamp for 3 minutes or so to form a pressure-sensitive adhesive layer having a thickness of about 100μm or so, thereby preparing a pressure-sensitive adhesive film. The punching factor (F) measured for the pressure-sensitive adhesive layer was 35.9 gf/μm.

## Example 2

A polymer component having a weight average amount (Mw) of about 85,000 was prepared in the same method as in Example 1, except that the types and ratios of the monomers used on the preparation of the polymer component were adjusted as shown in Table 4 below, and using the same, the pressure-sensitive adhesive layer and the pressure-sensitive adhesive film were prepared in the same manner. The punching factor (F) measured for the pressure-sensitive adhesive layer was about 68.4 gf/μm or so.

## Comparative Example 1

A polymer component having a weight average amount (Mw) of about 96,000 was prepared in the same method as in Example 1, except that the types and ratios of the monomers used on the preparation of the polymer component were adjusted as shown in Table 4 below, and using the same, the pressure-sensitive adhesive layer and the pressure-sensitive adhesive film were prepared in the same manner. The punching factor (F) measured for the pressure-sensitive adhesive layer was about 20.6 gf/μm or so.

## Comparative Example 2

A polymer component having a weight average amount (Mw) of about 100,000 was prepared in the same method as in Example 1, except that the types and ratios of the monomers used on the preparation of the polymer component were adjusted as shown in Table 4 below, and using the same, the pressure-sensitive adhesive layer and the pressure-sensitive adhesive film were prepared in the same manner. The punching factor (F) measured for the pressure-sensitive adhesive layer was about 22.8 gf/μm or so.

## Comparative Example 3

A polymer component having a weight average amount (Mw) of about 85,000 was prepared in the same method as in Example 1, except that the types and ratios of the monomers used on the preparation of the polymer component were adjusted as shown in Table 4 below, and using the same, the pressure-sensitive adhesive layer and the pressure-sensitive adhesive film were prepared in the same manner. The punching factor (F) measured for the pressure-sensitive adhesive layer was about 69.7 gf/μm or so.

## Comparative Example 4

A polymer component having a weight average amount (Mw) of about 41,000 was prepared in the same method as in Example 1, except that the types and ratios of the monomers used on the preparation of the polymer component were adjusted as shown in Table 4 below, and using the same, the pressure-sensitive adhesive layer and the pressure-sensitive adhesive film were prepared in the same manner. The punching factor (F) measured for the pressure-sensitive adhesive layer was about 6.4 gf/μm or so.

## Comparative Example 5

A polymer component having a weight average amount (Mw) of about 85,000 was prepared in the same method as in Example 1, except that the types and ratios of the monomers used on the preparation of the polymer component were adjusted as shown in Table 4 below, and using the same, the pressure-sensitive adhesive layer and the pressure-sensitive adhesive film were prepared in the same manner. The punching factor (F) measured for the pressure-sensitive adhesive layer was about 13.1 gf/μm or so.

## Comparative Example 6

A polymer component having a weight average amount (Mw) of about 109,000 was prepared in the same method as in Example 1, except that the types and ratios of the monomers used on the preparation of the polymer

component were adjusted as shown in Table 4 below, and using the same, the pressure-sensitive adhesive layer and the pressure-sensitive adhesive film were prepared in the same manner. The punching factor (F) measured for the pressure-sensitive adhesive layer was about 30.2 gf/μm or so.

[Table 4]

| (Unit: parts by weight) | | EHA | HEA | EHMA | mAAm | AAm | IBoA |
|---|---|---|---|---|---|---|---|
| Example | 1 | 80 | 6 | 7 | - | 4 | 3 |
| | 2 | 80 | 4 | 7 | - | 6 | 3 |
| Comparative Example | 1 | 74 | 8 | 11 | 4 | - | 3 |
| | 2 | 70 | 14 | 9 | 4 | - | 3 |
| | 3 | 78 | 6 | 7 | - | 6 | 3 |
| | 4 | 78 | 6 | 7 | - | 6 | 3 |
| | 5 | 82 | 6 | 7 | - | 2 | 3 |
| | 6 | 80 | 6 | 7 | - | 4 | 3 |
| *EHA: 2-Ethylhexyl acrylate<br>*HEA: 2-Hydroxyethyl acrylate<br>*EHMA: 2-Ethylhexyl methacrylate<br>*mAAm: Methacrylamide<br>*AAm: Acrylamide<br>*IBoA: Isobornyl acrylate | | | | | | | |

[Table 5]

| (Unit: parts by weight) | | Polymer component | 1651 | BPMA | SUO-1020 | HDDA | KBM403 |
|---|---|---|---|---|---|---|---|
| Example | 1 | 100 | 0.23 | 0.06 | 0.2 | 0.01 | 0.2 |
| | 2 | 100 | 0.23 | 0.06 | 0.2 | 0.01 | 0.2 |
| Comparative Example | 1 | 100 | 0.23 | 0.06 | 0.2 | 0.01 | 0.2 |
| | 2 | 100 | 0.23 | 0.06 | 0.2 | 0.01 | 0.2 |
| | 3 | 100 | 0.23 | 0.06 | 0.2 | 0.01 | 0.2 |
| | 4 | 100 | 0.23 | 0.06 | 0.2 | 0.01 | 0.2 |
| | 5 | 100 | 0.23 | 0.06 | 0.2 | 0.01 | 0.2 |
| | 6 | 100 | 0.23 | 0.06 | 0.20 | - | 0.2 |
| *1651: Ciba Specialty Chemicals<br>*BPMA: 4-Benzoylphenyl methacrylate<br>*SUO-1020: SHIN-A T&C<br>*HDDA: 1,6-Hexanediol diacrylate<br>*KBM403: Shin-Etsu | | | | | | | |

The evaluation results for Examples and Comparative Examples were summarized and described in Table 6 below.

[Table 6]

| | | White turbidity | Step difference | Tensile strength (Unit: gf) | Dielectric constant (@100KHz) |
|---|---|---|---|---|---|
| Example | 1 | O | ◎ | O (523) | 3.65 |
| | 2 | O | O | ◎ (751) | 3.52 |
| Comparative Example | 1 | X | O | Δ (377) | 3.81 |
| | 2 | X | X | Δ (383) | 4.19 |
| | 3 | O | Δ | ◎ (842) | 3.46 |
| | 4 | O | ◎ | X (204) | 3.60 |
| | 5 | Δ | O | Δ (279) | 3.47 |
| | 6 | O | ◎ | Δ (402) | 3.67 |

As identified from the results of Tables 4 and 6, it can be seen that the curable compositions (pressure-sensitive adhesive compositions) of Examples prevent white turbidity, have excellent tensile properties and exhibit low dielectric constants, while simultaneously securing appropriate punching properties and step absorptivity, which are opposite to each other.

On the other hand, in the case of Comparative Examples 1, 2, 4 and 5, the tensile strength was lowered, so that it could be expected that the punching properties would decrease, and in Comparative Example 3, the step absorption performance was lowered.

In Comparative Example 6, the tensile properties are lowered, so that it can be expected that the punching properties will decrease.

## Claims

1. A curable composition, comprising:

   a polymer component; and
   a curing agent,

   wherein the polymer component comprises an alkyl acrylate unit having a straight or branched alkyl group, a hydroxy group-containing monomer unit and a nitrogen-containing monomer unit, and
   wherein the curable composition has a punching factor in a range of 25 to 100 gf/μm.

2. The curable composition according to claim 1, wherein the curable composition has the maximum tensile strength before or after curing in a range of greater than 250 gf and 600 gf or less.

3. The curable composition according to claim 1, wherein the curable composition has the dielectric constant of 4.0 or less at a frequency of 100 kHz before or after curing.

4. The curable composition according to claim 1, wherein the hydroxy group-containing monomer is a compound of Formula 1 below:

[Formula 1]

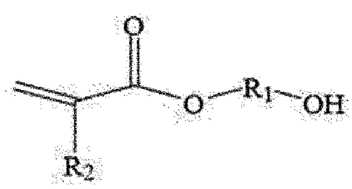

wherein, $R_1$ is an alkylene group, and R2 is hydrogen or an alkyl group.

5. The curable composition according to claim 1, wherein the polymer component comprises the hydroxy group-containing monomer unit and the nitrogen-containing monomer unit in an amount of 8 to 20 parts by weight relative to 100 parts by weight of the alkyl acrylate unit.

6. The curable composition according to claim 5, wherein the polymer component comprises the nitrogen-containing monomer unit in an amount of 2 to 10 parts by weight relative to 100 parts by weight of the alkyl acrylate unit.

7. The curable composition according to claim 5, wherein a weight ratio (B/A) of the hydroxy group-containing monomer unit (B) to the nitrogen-containing monomer unit (A) is in a range of 0.1 to 5.

8. The curable composition according to claim 1, wherein the nitrogen-containing monomer is one or more selected from the group consisting of (meth)acrylamide, N-alkyl (meth)acrylamide, N,N-dialkyl (meth)acrylamide, 1-aminobut-3-en-2-one, 1-(dimethylamino)but-3-en-2-one, 1-(dimethylamino)-3-methylbut-3-en-2-one, 5-aminopent-1-en-3-one, 5-(dimethylamino)pent-1-en-3-one, 5-(dimethylamino)-2-methylpent-1-en-3-one, 5-amino-2-methylpent-1-en-3-one and 5-amino-2-methylhex-1-en-3-one.

9. The curable composition according to claim 1, wherein the polymer component further comprises an alkyl methacrylate unit.

10. The curable composition according to claim 1, wherein the polymer component further comprises a unit of a compound of the following formula 2:

[Formula 2]

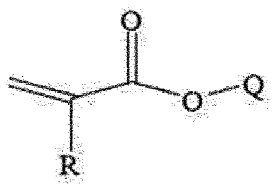

wherein, R is hydrogen or an alkyl group, and Q is a monovalent substituent having a non-aromatic ring structure with 3 to 20 carbon atoms.

11. The curable composition according to claim 1, wherein the polymer component comprises polymers in a range of 60 to 80 weight%.

12. The curable composition according to claim 1, wherein the polymer component has a weight average molecular weight (Mw) in a range of 50,000 to 300,000.

13. The curable composition according to claim 1, wherein the curing agent comprises two or more selected from the group consisting of a polyfunctional acrylate having a molar mass of 500 g/mol or less, a polyfunctional isocyanate compound and a polyfunctional urethane acrylate.

14. The curable composition according to claim 1, wherein the curing agent is included in a range of 0.01 to 10 parts by weight relative to 100 parts by weight of the polymer component.

15. The curable composition according to claim 1, wherein the curing agent comprises a polyfunctional acrylate having a molar mass of 500 g/mol or less and a polyfunctional urethane acrylate.

16. The curable composition according to claim 15, wherein the polyfunctional urethane acrylate is included in a ratio within the range of 0.01 parts by weight to 10 parts by weight relative to 100 parts by weight of the polymer component, where the weight ratio (C/D) of the polyfunctional urethane acrylate (C) to the polyfunctional acrylate (D) having a molar mass of 500 g/mol or less is in a range of 5 to 50.

17. The curable composition according to claim 1, further comprising a compound of the following formula 3:

[Formula 3]

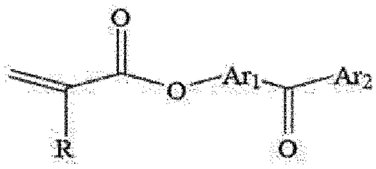

wherein, R is hydrogen or an alkyl group, An is an arylene group, and Ar2 is an aryl group.

**18.** A display device comprising the curable composition of claim 1 or a cured product thereof.

Figure 1

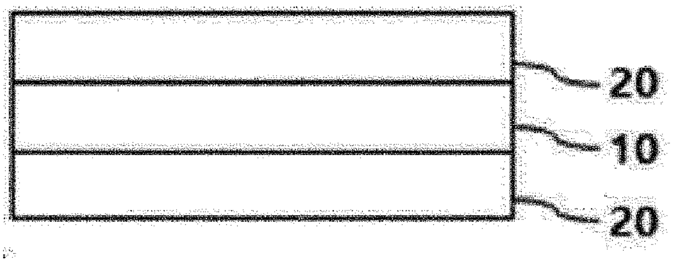


Figure 2

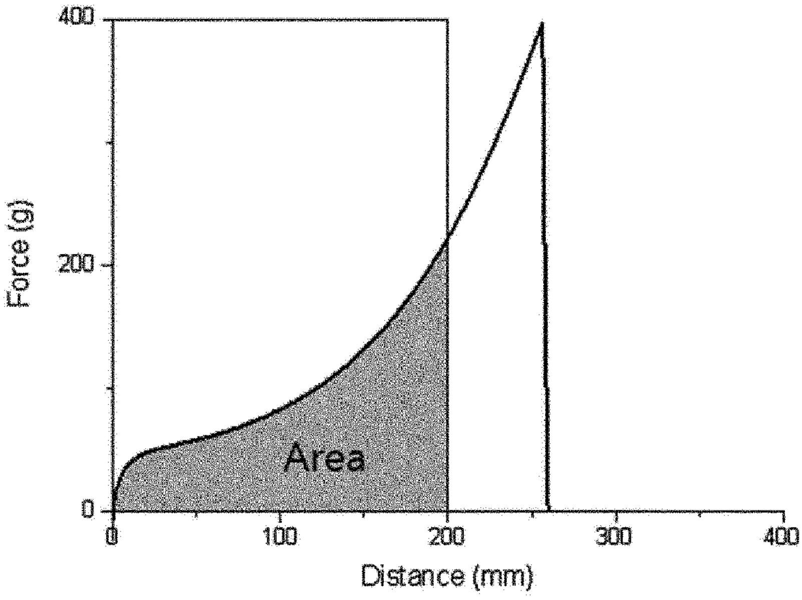


Figure 3

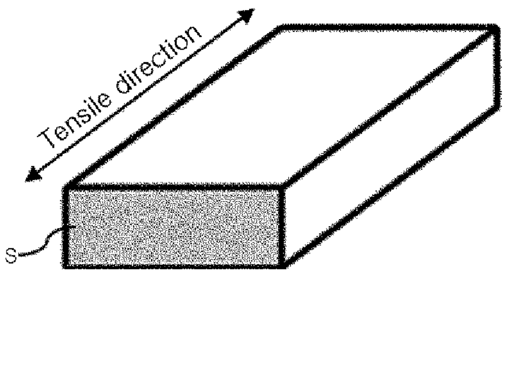

**INTERNATIONAL SEARCH REPORT**

International application No. **PCT/KR2020/014156**

**A. CLASSIFICATION OF SUBJECT MATTER**

**C09J 133/04**(2006.01)i; **C09J 7/38**(2018.01)i; **C09J 11/06**(2006.01)i

According to International Patent Classification (IPC) or to both national classification and IPC

**B. FIELDS SEARCHED**

Minimum documentation searched (classification system followed by classification symbols)

C09J 133/04(2006.01); C09D 11/30(2014.01); C09J 4/02(2006.01); C09J 7/00(2006.01); C09J 7/20(2018.01); G06F 3/041(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, Caplus), Google & keywords: 아크릴레이트(acrylate), 경화제(curing agent), 히드록시기(hydroxyl group), 디스플레이(display)

**C. DOCUMENTS CONSIDERED TO BE RELEVANT**

| Category* | Citation of document, with indication, where appropriate, of the relevant passages | Relevant to claim No. |
|---|---|---|
| X | KR 10-2014-0012623 A (HITACHI CHEMICAL COMPANY, LTD.) 03 February 2014 (2014-02-03) See claims 1, 6, 7 and 11-14; paragraphs [0018], [0036], [0044], [0052]-[0054], [0131]-[0132] and [0139]-[0147]; and table 1. | 1-12,18 |
| Y | | 13-17 |
| Y | KR 10-2017-0082190 A (LG CHEM, LTD.) 14 July 2017 (2017-07-14) See claims 5, 6 and 11; and paragraph [0048]. | 13-17 |
| A | KR 10-1882560 B1 (SAMSUNG SDI CO., LTD.) 26 July 2018 (2018-07-26) See claims 16-28. | 1-18 |
| A | KR 10-2018-0054725 A (KJ CHEMICALS CORPORATION) 24 May 2018 (2018-05-24) See claims 1-14. | 1-18 |
| A | KR 10-2016-0033307 A (LG HAUSYS, LTD.) 28 March 2016 (2016-03-28) See claims 1-14 and 16. | 1-18 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **21 January 2021** | **21 January 2021** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**
**Information on patent family members**

International application No.
**PCT/KR2020/014156**

| Patent document cited in search report | Publication date (day/month/year) | Patent family member(s) | Publication date (day/month/year) |
|---|---|---|---|
| KR 10-2014-0012623 A | 03 February 2014 | CN 103249797 A | 14 August 2013 |
| | | CN 103249797 B | 25 November 2015 |
| | | CN 105176419 A | 23 December 2015 |
| | | JP 2016-117883 A | 30 June 2016 |
| | | JP 5842825 B2 | 13 January 2016 |
| | | TW 201231602 A | 01 August 2012 |
| | | TW I534233 B | 21 May 2016 |
| | | WO 2012-077807 A1 | 14 June 2012 |
| KR 10-2017-0082190 A | 14 July 2017 | None | |
| KR 10-1882560 B1 | 26 July 2018 | CN 106550604 A | 29 March 2017 |
| | | CN 106550604 B | 16 June 2020 |
| | | JP 2016-035046 A | 17 March 2016 |
| | | JP 6688019 B2 | 28 April 2020 |
| | | TW 201610051 A | 16 March 2016 |
| | | TW I582190 B | 11 May 2017 |
| | | WO 2016-018003 A1 | 04 February 2016 |
| KR 10-2018-0054725 A | 24 May 2018 | CN 108350141 A | 31 July 2018 |
| | | EP 3351575 A1 | 25 July 2018 |
| | | JP 6277383 B2 | 14 February 2018 |
| | | TW 201720850 A | 16 June 2017 |
| | | TW I683832 B | 01 February 2020 |
| | | US 10676561 B2 | 09 June 2020 |
| | | US 2018-0244831 A1 | 30 August 2018 |
| | | WO 2017-047615 A1 | 23 March 2017 |
| | | WO 2017-047615 A1 | 28 September 2017 |
| KR 10-2016-0033307 A | 28 March 2016 | TW 201615790 A | 01 May 2016 |
| | | WO 2016-043521 A1 | 24 March 2016 |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 1020190128580
- KR 1020190128581